# EUROPEAN PATENT SPECIFICATION

(11) **EP 3 228 353 B1**
(45) Date of publication and mention of the grant of the patent: **12.06.2019**
(21) Application number: 17162409.1
(22) Date of filing: 22.03.2017
(51) Int. Cl.: A61N 1/36, A61H 39/00, A61N 1/04

(54) **UNIT FOR THE CORRECTION OF THE SUPPORTING-LOCOMOTIVE APPARATUS BY MICROCURRENT THERAPY**
EINHEIT ZUR KORREKTUR EINER HILFSLOKVORRICHTUNG DURCH MIKROSTROMTHERAPIE
UNITÉ DE CORRECTION DE L'APPAREIL LOCOMOTEUR DE SUPPORT PAR THÉRAPIE À MICRO-COURANT

(30) Priority: 04.04.2016 RU 2016112650
(43) Date of publication of application: 11.10.2017
(73) Proprietor: LLC "NeuroTechnology", Chelyabinsk 454021 (RU)
(72) Inventor: DENISENKO, Valeriy, 454020 Chelyabinsk (RU); KORYUKALOV, Yuriy, 454021 Chelyabinsk (RU)
(74) Representative: Spengler, Robert

(56) References cited:
- EP-A1- 3 205 324
- US-A1- 2005 131 462
- US-A1- 2009 259 151
- US-A1- 2015 112 405

## Description

### 1. Area of technology

This unit has been designed to be applied within the area of physiotherapy and is applicable to both medical and athletic therapeutic programs to provide correction of functional states of the supporting-locomotive apparatus to eliminate nervous overloads and fatigue. The unit also eliminates pelvic disorders and paravertebral muscle disorders, and restores muscle balance while providing therapeutic relief to the pelvis, the sternum, and the skull.

### 2. Invention background

There exist a wide range of efficient therapeutic means and techniques to restore the good functional state of the nervous system, the spine, and the overall human health.

Document EP 3 205 324 A1 discloses a unit for the action on biologically active body points and the relief of paravertebral muscles. The unit is designed as a base fitted with two pairs of narrow massage protrusions complete with spikes to correct paravertebral muscles of the thoracic and the lumbar segments, and a pair of wide protrusions for acupuncture action on the cervical region and bioactive body points. The protrusions are shaped as pyramids with rounded tops, with hollows between the protrusions for vertebral spinous processes to fit into, and the spacing between the protrusion pairs are the narrowest in the acupuncture neck massage protrusions and the widest between the lumbar massage protrusions. The acupuncture pair is fitted with spikes.

"The unit for the correction of the spine and paravertebral muscles" (holds Russian Federation patent No 2465881) and "The unit for the invigoration of the spine and the relief of paravertebral muscles" (holds Russian Federation patent No 144668) are the closest analogs to the unit. Each of the analogs in question is fitted with three pairs of massage protrusions to relieve the cervical, the thoracic, and the lumbar regions respectively. The fact that the massage protrusions of the latter two units were designed to work on the three spinal regions and not designed to work on the pelvis and the skull
can be viewed as a substantial design defect. This unit has been fitted with three supplementary pairs of acupuncture spikes to act on the muscle-ligamentous systems of both the spine and the intercostals muscles, as well as biologically active points of the sacrum and the skull. The spikes provide deeper targeting of trigger areas of the pelvis and the skull.

In marked contrast to the analogs, the unit is fitted with paired electrodes installed inside the spikes to provide relaxation of the functional state of the nervous system through both the lowermost region of the nervous system and immediately through the skull. In addition to nervous system correction through the paired electrodes, the unit can exert other types of therapeutic action on paravertebral muscles and bioactive body points.

### 3. Invention disclosure

It was the principle inventive problem to design a unit for the correction of the supporting-locomotive apparatus through the sacrum and the skull that are its two principal segments, as well as for the reflex regulation by acting on bioactive body points, and for deep action on muscle-ligamentous system of the biomechanical key segments of the supporting-locomotive apparatus to eliminate tensions within those segments and to restore muscle balance.

"The unit for the correction of the spine and paravertebral muscles" (holds Russian Federation patent No 2465881) and "The unit for the invigoration of the spine and the relief of paravertebral muscles" (holds Russian Federation patent No 144668) are the closest analogs to the unit. Each of the analogs in question is fitted with three pairs of massage protrusions to relieve the cervical, the thoracic, and the lumbar regions respectively. The fact that the massage protrusions of the latter two units were designed to work on the three spinal regions and not designed to work on the pelvis and the skull can be viewed as a substantial design defect. This unit has been fitted with three supplementary pairs of acupuncture spikes to act on the muscle-ligamentous systems of both the spine and the intercostals muscles, as well as biologically active points of the sacrum and the skull. The spikes provide deeper targeting of trigger areas of the pelvis, the thoracic spinal segment, the knees, and the skull.

Due to the absence of mechanisms for the correction of the nervous system, those units fail to provide complex rehabilitation to supporting-locomotive problem sufferers, as the problems in question are largely attributed to the state of the nervous system. By comparison, those defects in the latter units prevent safe and efficient action on the key segments of the supporting-locomotive apparatus and sufficient application to relaxation of the spine, the pelvis, and the skull.

In the light of the above, we have eliminated the defects by designing a unit for the correction of the supporting-locomotive apparatus by microcurrent therapy, shaped like an oblong base similar to a triangle, with protrusions of dissimilar widths located on the working surface, within the areas adjacent to one of the sides and to the opposite side of the triangle with protrusions of dissimilar widths, with spacings to fit to one of the segment of the supporting-locomotive apparatus for its correction. The triangular shape of the base has been designed to fit to the triangular shape of the sacrum and to provide relief to the sacrum in the most natural way, which the analogs to the unit fail to achieve.

Acupuncture spikes to act on biologically active points are located along the other two sides of the triangular base, that is, along the sides of the working surface of the unit.

The base of the unit has a flat bottom to provide stability when the unit under the body weight. There is a groove to fit to the spinous processes of the spine when the unit is being applied to the sacral region or to the thoracic region of the spine, with the working surface being flat and slightly caved in the middle, where the groove is.

The electrodes installed on the working surface or inside the spikes of the unit provide complex correction of the supporting-locomotive apparatus through the nervous system also - microcurrent therapy.

In addition to the correction of the nervous system through the paired electrodes, paravertebral muscles and biologically active points can receive other correction types, including vibrations, magnetic fields, sound, heat, and light.

### 4. Embodiment

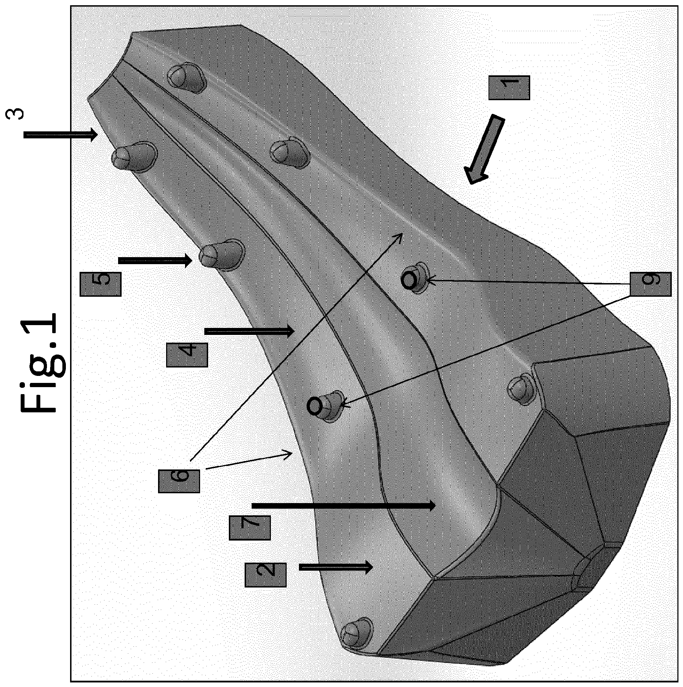

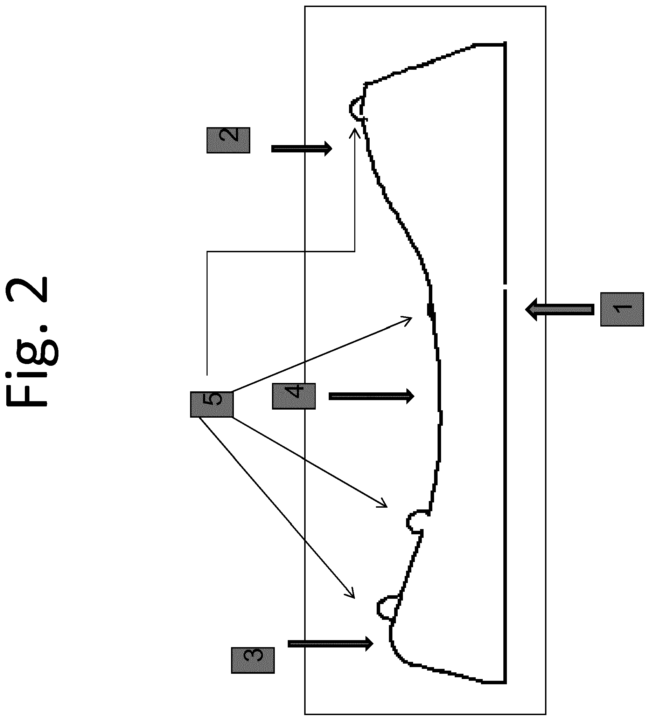

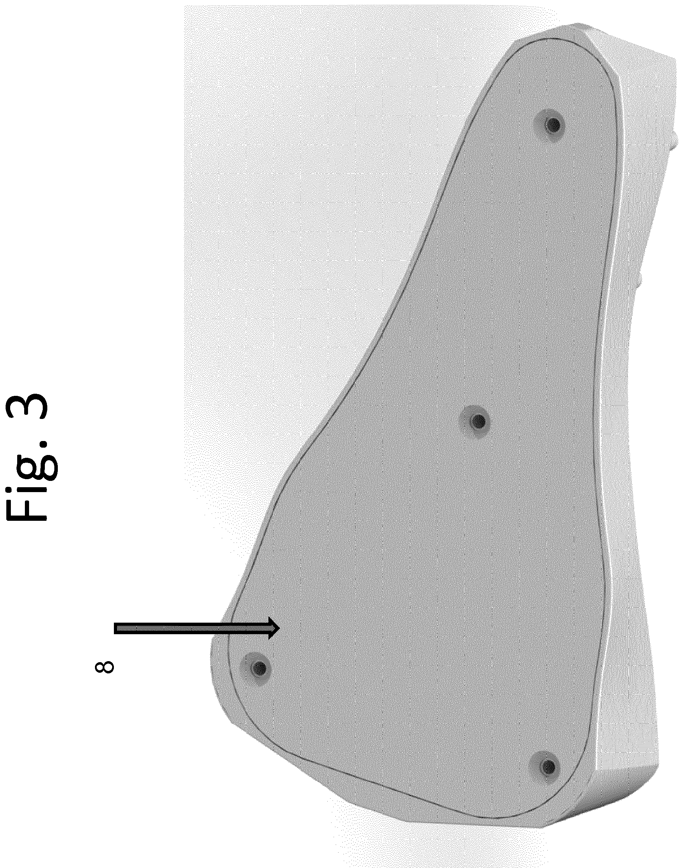

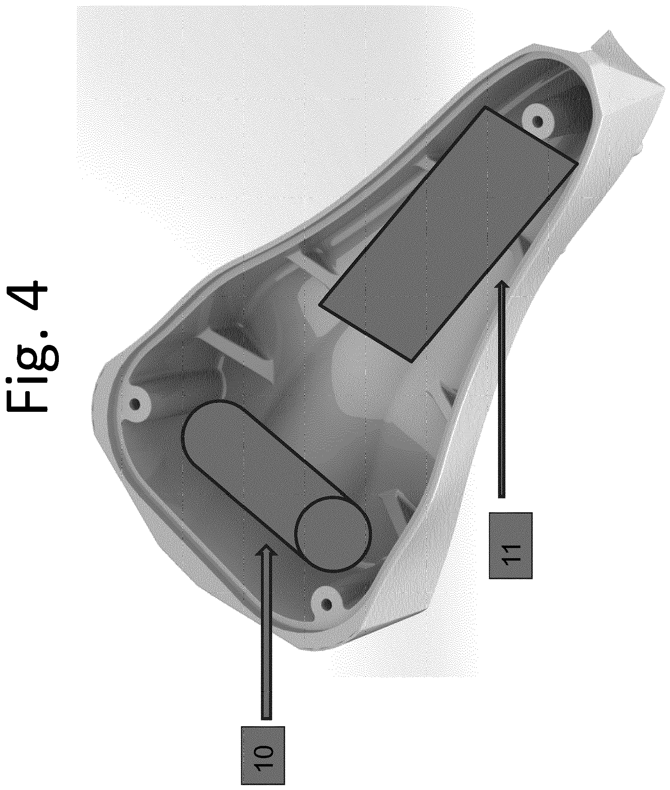

Fig 1 gives a top view and a side view of the unit; Fig 2 gives a side view of the unit; Fig 3 gives a bottom view of the unit with the cover on; and Fig 4 gives a bottom view and a view of the electronic module and the battery compartment with the cover off.

The unit for the correction of the supporting-locomotive apparatus by microcurrent therapy comprises the base (1), the protrusions on the wider side (2), the narrower side (3), which is the vertex of the triangular base, and a hollow (4) in between to fit to the sacrum, the thoracic region of the spine, the sternum, the knees, and the skull; the spikes (5) installed along the sides (6) of the work surface, and a tapered groove (7) running across the middle of the work surface (the upper side) of the unit to fit to the spinous processes of vertebrae (see Fig 1 and 2).

Fig 1 and 2 show the spikes (5) installed along the work surface of the unit to provide deeper targeting of trigger areas and bioactive points of the pelvis, the thoracic region of the spine, the knees, and the skull.

The cover (8) is in the bottom of the base (see Fig 3), with the width of the unit tapering from protrusion (2) to protrusion (3) (see Fig 1) to fit to the anatomic shape of the sacrum.

Fig 4 shows the inside of the unit with the battery compartment (10) and the electronic module (11) wired to the electrodes (9) (see Fig 1) through which the nervous system receives correction with body currents, while muscles and ligaments receive other correction types, including vibrations, sound, heat, light, and magnetic field, due to capabilities of modern combined electrodes and a vibration motor or a drive installed on the electronic module that provide deeper muscle relaxation and improve the functional state of the supporting-locomotive apparatus as a whole. The invention is set out in the appended claims.

### Exemplary use of the unit

The user should sit down and then lay down onto the unit so that the user's problem area of the supporting-locomotive apparatus (be it the pelvis, the thoracic region of the spine, or the skull) fit to the unit, the unit being positioned along the axis of the spine. The user should remain seated and fit either knee to the unit if they apply the unit to the knees, or fit the sacrum or the skull between protrusions 2 and 2 of the unit. As soon as the user has duly positioned the unit under the problem area of their supporting-locomotive apparatus, the user should remain lying on the working unit from 2 to 10 min, depending on the state of the specific area of their supporting-locomotive apparatus. The session works as acupuncture on a specific area of the supporting-locomotive apparatus, rendering tense segments of the muscle-ligamentous system into relaxation phase as the body weight presses on the unit, providing biomechanical relief to the spine, the skull, and other areas of the supporting-locomotive apparatus and restored balance between various muscle groups.

The spikes (5) can be used to provide acupuncture relief to bioactive points of the pelvis, the thoracic region of the spine, the knees, the skull, and other body areas. The electrodes (9) relax the functional state of the nervous system and facilitate muscle relaxation in a selected segment of the supporting-locomotive apparatus, largely due to the programs implemented by means of the electronic module installed inside the unit.

Relaxed sacral area and the skull, restored muscle balance in the supporting-locomotive apparatus, the action of the body weight, the acupuncture action of the spikes, and neurophysiologic action of the unit on key biomechanical segments of the supporting-locomotive apparatus eliminate of pathological blocks from the supportive-locomotive blocks and restore its normal functional state, while action on bioactive points activates regulatory processes in the body.

The user can give themselves one or more 10-to-30 min-long sessions a day, depending on the functional state of their supporting-locomotive apparatus and the whole body.

The unit enhances the efficiency of supporting-locomotive disorders and dysfunctions through microcurrent correction and due to the choice of appropriate body positions and poses, combined exercise with the unit, and activation of restorative processes; the unit provides instant pain relief, enhances the spinal column movement ease and amplitude, and speeds up regenerative processes.

The unit has been designed to take into account the user's functional state, and is easy to use, allowing the user to choose the impact and the session time, with no medical staff involved.

The unit allows preventing spinal disorders, pelvic disorders, knee disorders, and postural disorder.

### 5. Industrial applicability

The application areas of the unit include medicinal institutions (including healthcare institutions, preventive medicine institutions, and outpatient care), fitness centers, health centers, athletic facilities, and a domestic environment - as a basis of correctional, therapeutic, and rehabilitative sessions.

## Claims

1. A unit for the correction of the supporting-locomotive apparatus by microcurrent therapy comprising an oblong base (1) designed in the shape of a triangle, having protrusions of dissimilar widths located on the working surface at the vortex (3) and at the base (2) of the triangle, having a hollow (4) configured to fit to the sacrum, the thoracic region of the spine, the sternum, the knees, and the skull, the oblong base further comprising acupuncture spikes (5) configured to act on biologically active points and located along the other two sides of the triangular base (1), that is, along the sides (6) of the working surface of the unit, the oblong base further comprising battery-operated electrodes (9) installed on the working surface or inside the spikes (5) of the unit and wired to an electronic module (11) configured to provide heat and microcurrent therapy by skin contact.

2. The unit of claim 1 further comprising a groove (7) configured to fit to the spinous processes of the spine at the sacral region or the thoracic region.

## Patentansprüche

1. Das Gerät für die Einstellung des Bewegungsapparates mit Mikrostromtherapie, das aus dem ausgezogenen dreieckigen Gestell (1) entsteht, mit den Absätzen auf der Oberfläche und den Kanten des dreieckigen Gestells (3) mit der Höhlung (4), die in der Form des Kreuzbeines, der Brustwirbelsäule, des Brustbeines, der Knien und des Schädels angefertigt ist; im ausgezogenen Gestell gibt es weitere Akupressur-Dome (5), die mit dem Ziel der Wirkung auf biologisch aktiven Stippchen ausgearbeitet sind und die zwei Kanten des dreieckigen Gestells (1) entlang, d.h. die Seiten (6) der Arbeitsoberfläche des Geräts entlang, angeordnet sind; im ausgezogenen Gestell sind zusätzlich die Elektroden eingestellt, die auf die Oberfläche (9) durch Akupressur-Dome (5) herausgeführt sind und sich mit dem elektronischen Baustein (11) verbindet sind, der eine thermische und Mikrostromtherapie bei Hautkontakt bietet.

2. Das Gerät nach Anspruch 1, das die zusätzliche Höhlung (7) enthält, ist mit der Möglichkeit ausgearbeitet, es zu den Dornfortsätzen der Wirbelsäule in der Abteilungen der Kreuzwirbelsäule und Brustwirbelsäule anzupassen.

## Revendications

1. Appareil pour la correction du système musculo-squelettique avec la thérapie à micro courant qui se compose d'une base oblongue (1) de la forme triangulaire avec des ergots, qui se trouvent sur la surface et les facettes de la base triangulaire (3), qui a une cavité (4), faite en forme de sacrum, de rachis dorsal, de sternum, de genoux et de crâne; dans la base oblongue se trouvent les chevilles supplémentaires d'acupression (5), élaborées afin d'exécuter l'action sur les points biologiquement actifs et situeés le long de deux facettes de la base triangulaire (1), c'est-à-dire le long des côtés (6) de la surface fonctionnelle de l'appareil; en plus dans la base oblongue sont installeés les électrodes, qui sont portées (9) sur la surface fonctionnelle à travers les chevilles d'acupression (5) et sont branchées au module électronique (11), qui assure la thérapie thermique et celle à micro courant au contact avec la peau.

2. Appareil du r.1 qui comprend une cavité supplémentaire (7), élaboré avec la possibilité de raccordement aux apophyses épinaires de l'épine dorsale dans le rachis dorsal ou dans le rachis sacré.
